# EUROPÄISCHE PATENTSCHRIFT

(11) **EP 0 786 004 B1**
(45) Veröffentlichungstag und Bekanntmachung des Hinweises auf die Patenterteilung: **08.08.2001**
(21) Anmeldenummer: 95936464.7
(22) Anmeldetag: 10.10.1995
(51) Int. Cl.: C12N 15/54, C12N 9/12, C12N 15/62, A61K 48/00, C07K 16/40, A61K 38/43, A61K 39/395, C12Q 1/48

(54) **KLONIERUNG, EXPRESSION UND CHARAKTERISIERUNG EINER NEUEN FORM DER PHOSPHATIDYLINOSITOL-3-KINASE**
CLONING, EXPRESSION AND CHARACTERISATION OF A NOVEL FORM OF PHOSPHATIDYLINOSITOL-3-KINASE
CLONAGE, EXPRESSION ET CARACTERISATION D'UNE NOUVELLE FORME DE PHOSPHATIDYLINOSITOL-3-KINASE

(30) Priorität: 13.10.1994 DE 4436696; 20.12.1994 DE 4445562
(43) Veröffentlichungstag der Anmeldung: 30.07.1997
(73) Patentinhaber: Max-Planck-Gesellschaft zur Förderung der Wissenschaften e.V., 80539 München (DE)
(72) Erfinder: STOYANOV, Borislav, D-07778 Dorndorf (DE); HANCK, Theodor, D-07743 Jena (DE); WETZKER, Reinhard, D-07747 Jena (DE)
(74) Vertreter: Weiss, Wolfgang, Dipl.-Chem. Dr.
(86) Internationale Anmeldenummer: EP9503990
(87) Internationale Veröffentlichungsnummer: WO9612024

(56) Entgegenhaltungen:
- SCIENCE, AUG 4 1995, 269 (5224) P690-3, UNITED STATES, STOYANOV B ET AL 'Cloning and characterization of a G protein-activated human phosphoinositide-3 kinase.'
- MOLECULAR AND CELLULAR BIOLOGY, Bd. 13, Nr. 12, Dezember 1993 WASHINGTON US, Seiten 7677-7688, HU P. ET AL. 'Cloning of a novel, ubiquitously expressed human phosphatidylinositol 3-kinase and identification of its bindin site on p85' in der Anmeldung erwähnt
- CELL, APR 8 1994, 77 (1) P83-93, UNITED STATES, STEPHENS L ET AL 'A novel phosphoinositide 3 kinase activity in myeloid-derived cells is activated by G protein beta gamma subunits.' in der Anmeldung erwähnt

### SEQUENZPROTOKOLLE

(1) ALGEMEINE INFORMATION:
   (i) ANMELDER:
      (A) NAME: Max-Planck-Gesellschaft zur Foerderung der Wissenschaften
      (B) STRASSE: Hofgartenstr. 2
      (C) ORT: Muenchen
      (E) LAND: DE
      (F) POSTLEITZAHL: 80539
   (ii) ANMELDETITEL: Klonierung, Expression und Charakterisierung einer neuen Form der Phosphatidylinositol-3-Kinase
   (iii) ANZAHL DER SEQUENZEN: 4
   (iv) COMPUTER-LESBARE FORM:
      (A) DATENTR GER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 4134 Basenpaare
      (B) ART: Nukleinsäure
      (C) STRANGFORM: beides
      (D) TOPOLOGIE: linear
   (ii) ART DES MOLEKÜLS: cDNS
   (vi) URSPRÜNGLICHE HERKUNFT:
      (A) ORGANISMUS: Homo sapiens
   (ix) MERKMALE:
      (A) NAME/SCHLÜSSEL: CDS
      (B) LAGE: 423..3569
   (xi) SEQUENZBESCHREIBUNG: SEQ ID NO: 1:
   GAATTCGGCA CGAGCACTTC CTTCTCGGCT AGATTATCTG AAACTGTTGT CGGTTCTTGA      60
   GATGATACTA CCACCGAATG TCTGTGTTTC ATTGTCTAGT CCAACCTGTA TTGTGGATAT     120
   CTACAACGTT CCGGCAATAG TTTTGCAGGT GCATCACATT TTTGTTTTTG TTTTGGGAGG     180
   AAAAGGGAGG GCACGGCAGC CAGGCTTCAT ATTCCTACAA GTGCATGCTT CAAGATTACT     240
   GTACTTACAG TGTTTCCAAC ATCTTCTCAT AAAAGGGGAA AGCTTCATAG CCTCAACCAT     300
   GAAGGAAACC AGTCGCATAG GGCATGGAGC TGGAGAACTA TAAACAGCCC GTGGTGCTGA     360
   GAGAGGACAA CTGCCGAAGG CGCCGGAGGA TGAAGCCGCG CAGTGCTGCC AGCCTGTCCT     420
   CC ATG GAG CTC ATC CCC ATC GAG TTC GTG CTG CCC ACC AGC CAG CGC        467
      Met Glu Leu Ile Pro Ile Glu Phe Val Leu Pro Thr Ser Gln Arg
       1               5                  10                  15
   AAA TGC AAG AGC CCC GAA ACG GCG CTG CTG CAC GTG GCC GGC CAC GGC       515
   Lys Cys Lys Ser Pro Glu Thr Ala Leu Leu His Val Ala Gly His Gly
                   20                  25                  30
   AAC GTG GAG CAG ATG AAG GCC CAG GTG TGG CTG CGA GCG CTG GAG ACC       563
   Asn Val Glu Gln Met Lys Ala Gln Val Trp Leu Arg Ala Leu Glu Thr
               35                  40                  45
   AGC TGG CGC GGA CTT CTA CCA CCG GCT GGG ACC GCA TCA CTT CCT CCT      611
   Ser Trp Arg Gly Leu Leu Pro Pro Ala Gly Thr Ala Ser Leu Pro Pro
            50                  55                  60
   GCT CTA TCA GAA GAA GGG CAG TGG TAC GAG ATC TAC GAC AAG TAC CAG      659
   Ala Leu Ser Glu Glu Gly Gln Trp Tyr Glu Ile Tyr Asp Lys Tyr Gln
        65                  70                  75
   GTG GTG CAG ACT CTG GAC TGC CTG CGC TAC TGG AAG GCC ACG CAC CGG      707
   Val Val Gln Thr Leu Asp Cys Leu Arg Tyr Trp Lys Ala Thr His Arg
    80                  85                  90                  95
   AGC CCG GGC CAG ATC CAC CTG GTG CAG CGG CAC CCG CCC TCC GAG GAG      755
   Ser Pro Gly Gln Ile His Leu Val Gln Arg His Pro Pro Ser Glu Glu
                   100                 105                 110
   TCC CAA GCC TTC CAG CGG CAG CTC ACG GCG CTG ATT GGC TAT GAC GTC      803
   Ser Gln Ala Phe Gln Arg Gln Leu Thr Ala Leu Ile Gly Tyr Asp Val
               115                 120                 125
   ACT GAC GTC AGC AAC GTG CAC GAC GAT GAG CTG GAG TTC ACG CGC CGT      851
   Thr Asp Val Ser Asn Val His Asp Asp Glu Leu Glu Phe Thr Arg Arg
           130                 135                 140
   GGC TTG GTG ACC CCG CGC ATG GCG GAG GTG GCC AGC CGC GAC CCC AAG      899
   Gly Leu Val Thr Pro Arg Met Ala Glu Val Ala Ser Arg Asp Pro Lys
       145                 150                 155
   CTC TAC GCC ATG CAC CCG TGG GTG ACG TCC AAG CCC CTC CCG GAG TAC      947
   Leu Tyr Ala Met His Pro Trp Val Thr Ser Lys Pro Leu Pro Glu Tyr
   160                 165                 170                 175
   CTG TGG AAG AAG ATT GCC AAC AAC TGC ATC TTC ATC GTC ATT CAC CGC      995
   Leu Trp Lys Lys Ile Ala Asn Asn Cys Ile Phe Ile Val Ile His Arg
                   180                 185                 190
   AGC ACC ACC AGC CAG ACC ATT AAG GTC TCA CCC GAC GAC ACC CCC GGC     1043
   Ser Thr Thr Ser Gln Thr Ile Lys Val Ser Pro Asp Asp Thr Pro Gly
               195                 200                 205
   GCC ATC CTG CAG AGC TTC TTC ACC AAG ATG GCC AAG AAG AAA TCT CTG     1091
   Ala Ile Leu Gln Ser Phe Phe Thr Lys Met Ala Lys Lys Lys Ser Leu
           210                 215                 220
   ATG GAT ATT CCC GAA AGC CAA AGC GAA CAG GAT TTT GTG CTG CGC GTC     1139
   Met Asp Ile Pro Glu Ser Gln Ser Glu Gln Asp Phe Val Leu Arg Val
       225                 230                 235
   TGT GGC CGG GAT GAG TAC CTG GTG GGC GAA ACG CCC ATC AAA AAC TTC     1187
   Cys Gly Arg Asp Glu Tyr Leu Val Gly Glu Thr Pro Ile Lys Asn Phe
   240                 245                 250                 255
   CAG TGG GTG AGG CAC TGC CTC AAG AAC GGA GAA GAG ATT CAC GTG GTA     1235
   Gln Trp Val Arg His Cys Leu Lys Asn Gly Glu Glu Ile His Val Val
                   260                 265                 270
   CTG GAC ACG CCT CCA GAC CCG GCC CTA GAC GAG GTG AGG AAG GAA GAG     1283
   Leu Asp Thr Pro Pro Asp Pro Ala Leu Asp Glu Val Arg Lys Glu Glu
               275                 280                 285
   TGG CCG CTG GTG GAC GAC TGC ACG GGA GTC ACC GGC TAC CAT GAG CAG     1331
   Trp Pro Leu Val Asp Asp Cys Thr Gly Val Thr Gly Tyr His Glu Gln
           290                 295                 300
   CTT ACC ATC CAC GGC AAG GAC CAC GAG AGT GTG TTC ACC GTG TCC CTG     1379
   Leu Thr Ile His Gly Lys Asp His Glu Ser Val Phe Thr Val Ser Leu
       305                 310                 315
   TGG GAC TGC GAC CGC AAG TTC AGG GTC AAG ATC AGA GGC ATT GAT ATC      1427
   Trp Asp Cys Asp Arg Lys Phe Arg Val Lys Ile Arg Gly Ile Asp Ile
   320                 325                 330                 335
   CCC GTC CTG CCT CGG AAC ACC GAC CTC ACA GTT TTT GTA GAG GCA AAC      1475
   Pro Val Leu Pro Arg Asn Thr Asp Leu Thr Val Phe Val Glu Ala Asn
                   340                 345                 350
   ATC CAG CAT GGG CAA CAA GTC CTT TGC CAA AGG AGA ACC AGC CCC AAA      1523
   Ile Gln His Gly Gln Gln Val Leu Cys Gln Arg Arg Thr Ser Pro Lys
               355                 360                 365
   CCC TTC ACA GAG GAG GTG CTG TGG AAT GTG TGG CTT GAG TTC AGT ATC      1571
   Pro Phe Thr Glu Glu Val Leu Trp Asn Val Trp Leu Glu Phe Ser Ile
           370                 375                 380
   AAA ATC AAA GAC TTG CCC AAA GGG GCT CTA CTG AAC CTC CAG ATC TAC      1619
   Lys Ile Lys Asp Leu Pro Lys Gly Ala Leu Leu Asn Leu Gln Ile Tyr
       385                 390                 395
   TGC GGT AAA GCT CCA GCA CTG TCC AGC AAG GCC TCT GCA GAG TCC CCC      1667
   Cys Gly Lys Ala Pro Ala Leu Ser Ser Lys Ala Ser Ala Glu Ser Pro
   400                 405                 410                 415
   AGT TCT GAG TCC AAG GGC AAA GTT CGG CTT CTC TAT TAT GTG AAC CTG      1715
   Ser Ser Glu Ser Lys Gly Lys Val Arg Leu Leu Tyr Tyr Val Asn Leu
                   420                 425                 430
   CTG CTG ATA GAC CAC CGT TTC CTC CTG CGC CGT GGA GAA TAC GTC CTC      1763
   Leu Leu Ile Asp His Arg Phe Leu Leu Arg Arg Gly Glu Tyr Val Leu
               435                 440                 445
   CAC ATG TGG CAG ATA TCT GGG AAG GGA GAA GAC CAA GGA AGC TTC AAT      1811
   His Met Trp Gln Ile Ser Gly Lys Gly Glu Asp Gln Gly Ser Phe Asn
           450                 455                 460
   GCT GAC AAA CTC ACG TCT GCA ACT AAC CCA GAC AAG GAG AAC TCA ATG      1859
   Ala Asp Lys Leu Thr Ser Ala Thr Asn Pro Asp Lys Glu Asn Ser Met
       465                 470                 475
   TCC ATC TCC ATT CTT CTG GAC AAT TAC TGC CAC CCG ATA GCC CTG CCT      1907
   Ser Ile Ser Ile Leu Leu Asp Asn Tyr Cys His Pro Ile Ala Leu Pro
   480                 485                 490                 495
   AAG CAT CAG CCC ACC CCT GAC CCG GAA GGG GAC CGG GTT CGA GCA GAA      1955
   Lys His Gln Pro Thr Pro Asp Pro Glu Gly Asp Arg Val Arg Ala Glu
                   500                 505                 510
   ATG CCC AAC CAG CTT CGC AAG CAA TTG GAG GCG ATC ATA GCC ACT GAT      2003
   Met Pro Asn Gln Leu Arg Lys Gln Leu Glu Ala Ile Ile Ala Thr Asp
               515                 520                 525
   CCA CTT AAC CCT CTC ACA GCA GAG GAC AAA GAA TTG CTC TGG CAT TTT      2051
   Pro Leu Asn Pro Leu Thr Ala Glu Asp Lys Glu Leu Leu Trp His Phe
           530                 535                 540
   AGA TAC GAA AGC CTT AAG CAC CCA AAA GCA TAT CCT AAG CTA TTT AGT      2099
   Arg Tyr Glu Ser Leu Lys His Pro Lys Ala Tyr Pro Lys Leu Phe Ser
       545                 550                 555
   TCA GTG AAA TGG GGA CAG CAA GAA ATT GTG GCC AAA ACA TAC CAA TTG      2147
   Ser Val Lys Trp Gly Gln Gln Glu Ile Val Ala Lys Thr Tyr Gln Leu
   560                 565                 570                 575
   TTG GCC AGA AGG GAA GTC TGG GAT CAA AGT GCT TTG GAT GTT GGG TTA      2195
   Leu Ala Arg Arg Glu Val Trp Asp Gln Ser Ala Leu Asp Val Gly Leu
                   580                 585                 590
   ACA ATG CAG CTC CTG GAC TGC AAC TTC TCA GAT GAA AAT GTA AGA GCC 2243
   Thr Met Gln Leu Leu Asp Cys Asn Phe Ser Asp Glu Asn Val Arg Ala
   595 600 605
   ATT GCA GTT CAG AAA CTG GAG AGC TTG GAG GAC GAT GAT GTT CTG CAT 2291
   Ile Ala Val Gln Lys Leu Glu Ser Leu Glu Asp Asp Asp Val Leu His
   610 615 620
   TAC CTT CTA CAA TTG GTC CAG GCT GTG AAA TTT GAA CCA TAC CAT GAT 2339
   Tyr Leu Leu Gln Leu Val Gln Ala Val Lys Phe Glu Pro Tyr His Asp
   625 630 635
   AGC GCC CTT GCC AGA TTT CTG CTG AAG CGT GGT TTA AGA AAC AAA AGA 2387
   Ser Ala Leu Ala Arg Phe Leu Leu Lys Arg Gly Leu Arg Asn Lys Arg
   640 645 650 655
   ATT GGT CAC TTT TTG TTT TGG TTC TTG AGA AGT GAG ATA GCC CAG TCC 2435
   Ile Gly His Phe Leu Phe Trp Phe Leu Arg Ser Glu Ile Ala Gln Ser
   660 665 670
   AGA CAC TAT CAG CAG AGG TTC GCT GTG ATT CTG GAA GCC TAT CTG AGG 2483
   Arg His Tyr Gln Gln Arg Phe Ala Val Ile Leu Glu Ala Tyr Leu Arg
   675 680 685
   GGC TGT GGC ACA GCC ATG CTG CAC GAC TTT ACC CAA CAA GTC CAA GTA 2531
   Gly Cys Gly Thr Ala Met Leu His Asp Phe Thr Gln Gln Val Gln Val
   690 695 700
   ATC GAG ATG TTA CAA AAA GTC ACC CTT GAT ATT AAA TCG CTC TCT GCT 2579
   Ile Glu Met Leu Gln Lys Val Thr Leu Asp Ile Lys Ser Leu Ser Ala
   705 710 715
   GAA AAG TAT GAC GTC AGT TCC CAA GTT ATT TCA CAA CTT AAA CAA AAG 2627
   Glu Lys Tyr Asp Val Ser Ser Gln Val Ile Ser Gln Leu Lys Gln Lys
   720 725 730 735
   CTT GAA AAC CTG CAG AAT TCT CAA CTC CCC GAA AGC TTT AGA GTT CCA 2675
   Leu Glu Asn Leu Gln Asn Ser Gln Leu Pro Glu Ser Phe Arg Val Pro
   740 745 750
   TAT GAT CCT GGA CTG AAA GCA GGA GCG CTG GCA ATT GAA AAA TGT AAA 2723
   Tyr Asp Pro Gly Leu Lys Ala Gly Ala Leu Ala Ile Glu Lys Cys Lys
   755 760 765
   GTA ATG GCC TCC AAG AAA AAA CCA CTA TGG CTT GAG TTT AAA TGT GCC 2771
   Val Met Ala Ser Lys Lys Lys Pro Leu Trp Leu Glu Phe Lys Cys Ala
   770 775 780
   GAT CCT ACA GCC CTA TCA AAT GAA ACA ATT GGA ATT ATC TTT AAA CAT 2819
   Asp Pro Thr Ala Leu Ser Asn Glu Thr Ile Gly Ile Ile Phe Lys His
   785 790 795
   GGT GAT GAT CTG CGC CAA GAC ATG CTT ATT TTA CAG ATT CTA CGA ATC 2867
   Gly Asp Asp Leu Arg Gln Asp Met Leu Ile Leu Gln Ile Leu Arg Ile
   800 805 810 815
   ATG GAG TCT ATT TGG GAG ACT GAA TCT TTG GAT CTA TGC CTC CTG CCA 2915
   Met Glu Ser Ile Trp Glu Thr Glu Ser Leu Asp Leu Cys Leu Leu Pro
   820 825 830
   TAT GGT TGC ATT TCA ACT GGT GAC AAA ATA GGA ATG ATC GAG ATT GTG 2963
   Tyr Gly Cys Ile Ser Thr Gly Asp Lys Ile Gly Met Ile Glu Ile Val
   835 840 845
   AAA GAC GCC ACG ACA ATT GCC AAA ATT CAG CAA AGC ACA GTG GGC AAC 3011
   Lys Asp Ala Thr Thr Ile Ala Lys Ile Gln Gln Ser Thr Val Gly Asn
   850 855 860
   ACG GGA GCA TTT AAA GAT GAA GTC CTG AAT CAC TGG CTC AAA GAA AAA      3059
   Thr Gly Ala Phe Lys Asp Glu Val Leu Asn His Trp Leu Lys Glu Lys
       865                 870                 875
   TCC CCT ACT GAA GAA AAG TTT CAG GCA GCA GTG GAG AGA TTT GTT TAT      3107
   Ser Pro Thr Glu Glu Lys Phe Gln Ala Ala Val Glu Arg Phe Val Tyr
   880                 885                 890                 895
   TCC TGT GCA GGC TAC TGT GTG GCA ACC TTT GTT CTT GGA ATA GGC GAC      3155
   Ser Cys Ala Gly Tyr Cys Val Ala Thr Phe Val Leu Gly Ile Gly Asp
                   900                 905                 910
   AGA CAC AAT GAC AAT ATT ATG ATC ACC GAG ACA GGA AAC CTA TTT CAT      3203
   Arg His Asn Asp Asn Ile Met Ile Thr Glu Thr Gly Asn Leu Phe His
               915                 920                 925
   ATT GAC TTC GGG CAC ATT CTT GGG AAT TAC AAA AGT TTC CTG GGC ATT      3251
   Ile Asp Phe Gly His Ile Leu Gly Asn Tyr Lys Ser Phe Leu Gly Ile
           930                 935                 940
   AAT AAA GAG AGA GTG CCA TTT GTG CTA ACC CCT GAC TTC CTC TTT GTG      3299
   Asn Lys Glu Arg Val Pro Phe Val Leu Thr Pro Asp Phe Leu Phe Val
       945                 950                 955
   ATG GGA ACT TCT GGA AAG AAG ACA AGC CCA CAC TTC CAG AAA TTT CAG      3347
   Met Gly Thr Ser Gly Lys Lys Thr Ser Pro His Phe Gln Lys Phe Gln
   960                 965                 970                 975
   GAC ATC TGT GTT AAG GCT TAT CTA GCC CTT CGT CAT CAC ACA AAC CTA      3395
   Asp Ile Cys Val Lys Ala Tyr Leu Ala Leu Arg His His Thr Asn Leu
                   980                 985                 990
   CTG ATC ATC CTG TTC TCC ATG ATG CTG ATG ACA GGA ATG CCC CAG TTA      3443
   Leu Ile Ile Leu Phe Ser Met Met Leu Met Thr Gly Met Pro Gln Leu
               995                 1000                1005
   ACA AGC AAA GAA GAC ATT GAA TAT ATC CGG GAT GCC CTC ACA GTG GGG      3491
   Thr Ser Lys Glu Asp Ile Glu Tyr Ile Arg Asp Ala Leu Thr Val Gly
           1010                1015                1020
   AAA AAT GAG GAG GAT GCT AAA AAG TAT TTT CTT GAT CAG ATC GAA GTT      3539
   Lys Asn Glu Glu Asp Ala Lys Lys Tyr Phe Leu Asp Gln Ile Glu Val
       1025                1030                1035
   TGG CAG AGA CAA AGG ATG GAC TGT GCA GTT TAATTGGTTT CTACATCTTG        3589
   Trp Gln Arg Gln Arg Met Asp Cys Ala Val
   1040                1045
   TTCTTGGCAT CAAACAAGGA GAGAAACATT CAGCCTAATA CTTTAGGCTA GAATCAAAAA    3649
   CAAGTTAGTG TTCTATGGTT TAAATTAGCA TAGCAATCAT CGAACTTGGA TTTCAAATGC    3709
   AATAGACATT GTGAAAGCTG GCATTTCAGA AGTATAGCTC TTTTCCTACC TGAACTCTTC    3769
   CCTGGAGAAA AGATGTTGGC ATTGCTGATT GTTTGGTTAA GCAATGTCCA GTGCTAGGAT    3829
   TATTTGCAGG TTTGGTTTTT TCTCATTTGT CTGTGGCATT GGAGAATATT CTCGGTTTAA    3889
   ACAGACTAAT GACTTCCTTA TTGTCCCTGA TATTTTGACT ATCTTACTAT TGAGTGCTTC    3949
   TGGAAATTCT TTGGAATAAT TGATGACATC TATTTTCATC TGGGTTTAGT CTCAATTTTG    4009
   GTTATCTTTG TGTTCCTCAA GCTCTTTAAA GAAAAAGATG TAATCGTTGT AACCTTTGTC    4069
   TCATTCCTTA AATGATGCTT CCAAACATCT CCTTAGTGTC TGCAGGTGTT AGTGGTGTGC    4129
   TAAAA                                                                4134
(2) INFORMATION ZU SEQ ID NO: 2:
   (i) SEQUENZ CHARAKTERISTIKA:
      (A) LÄNGE: 1049 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Glu Leu Ile Pro Ile Glu Phe Val Leu Pro Thr Ser Gln Arg Lys
    1               5                  10                  15
   Cys Lys Ser Pro Glu Thr Ala Leu Leu His Val Ala Gly His Gly Asn
               20                  25                  30
   Val Glu Gln Met Lys Ala Gln Val Trp Leu Arg Ala Leu Glu Thr Ser
           35                  40                  45
   Trp Arg Gly Leu Leu Pro Pro Ala Gly Thr Ala Ser Leu Pro Pro Ala
       50                  55                  60
   Leu Ser Glu Glu Gly Gln Trp Tyr Glu Ile Tyr Asp Lys Tyr Gln Val
   65                  70                  75                  80
   Val Gln Thr Leu Asp Cys Leu Arg Tyr Trp Lys Ala Thr His Arg Ser
                   85                  90                  95
   Pro Gly Gln Ile His Leu Val Gln Arg His Pro Pro Ser Glu Glu Ser
               100                 105                 110
   Gln Ala Phe Gln Arg Gln Leu Thr Ala Leu Ile Gly Tyr Asp Val Thr
           115                 120                 125
   Asp Val Ser Asn Val His Asp Asp Glu Leu Glu Phe Thr Arg Arg Gly
       130                 135                 140
   Leu Val Thr Pro Arg Met Ala Glu Val Ala Ser Arg Asp Pro Lys Leu
   145                 150                 155                 160
   Tyr Ala Met His Pro Trp Val Thr Ser Lys Pro Leu Pro Glu Tyr Leu
                   165                 170                 175
   Trp Lys Lys Ile Ala Asn Asn Cys Ile Phe Ile Val Ile His Arg Ser
               180                 185                 190
   Thr Thr Ser Gln Thr Ile Lys Val Ser Pro Asp Asp Thr Pro Gly Ala
           195                 200                 205
   Ile Leu Gln Ser Phe Phe Thr Lys Met Ala Lys Lys Lys Ser Leu Met
       210                 215                 220
   Asp Ile Pro Glu Ser Gln Ser Glu Gln Asp Phe Val Leu Arg Val Cys
   225                 230                 235                 240
   Gly Arg Asp Glu Tyr Leu Val Gly Glu Thr Pro Ile Lys Asn Phe Gln
                   245                 250                 255
   Trp Val Arg His Cys Leu Lys Asn Gly Glu Glu Ile His Val Val Leu
               260                 265                 270
   Asp Thr Pro Pro Asp Pro Ala Leu Asp Glu Val Arg Lys Glu Glu Trp
           275                 280                 285
   Pro Leu Val Asp Asp Cys Thr Gly Val Thr Gly Tyr His Glu Gln Leu
       290                 295                 300
   Thr Ile His Gly Lys Asp His Glu Ser Val Phe Thr Val Ser Leu Trp
   305                 310                 315                 320
   Asp Cys Asp Arg Lys Phe Arg Val Lys Ile Arg Gly Ile Asp Ile Pro
                   325                 330                 335
   Val Leu Pro Arg Asn Thr Asp Leu Thr Val Phe Val Glu Ala Asn Ile
               340                 345                 350
   Gln His Gly Gln Gln Val Leu Cys Gln Arg Arg Thr Ser Pro Lys Pro
           355                 360                 365
   Phe Thr Glu Glu Val Leu Trp Asn Val Trp Leu Glu Phe Ser Ile Lys
       370                 375                 380
   Ile Lys Asp Leu Pro Lys Gly Ala Leu Leu Asn Leu Gln Ile Tyr Cys
   385                 390                 395                 400
   Gly Lys Ala Pro Ala Leu Ser Ser Lys Ala Ser Ala Glu Ser Pro Ser
                   405                 410                 415
   Ser Glu Ser Lys Gly Lys Val Arg Leu Leu Tyr Tyr Val Asn Leu Leu
               420                 425                 430
   Leu Ile Asp His Arg Phe Leu Leu Arg Arg Gly Glu Tyr Val Leu His
           435                 440                 445
   Met Trp Gln Ile Ser Gly Lys Gly Glu Asp Gln Gly Ser Phe Asn Ala
       450                 455                 460
   Asp Lys Leu Thr Ser Ala Thr Asn Pro Asp Lys Glu Asn Ser Met Ser
   465                 470                 475                 480
   Ile Ser Ile Leu Leu Asp Asn Tyr Cys His Pro Ile Ala Leu Pro Lys
                   485                 490                 495
   His Gln Pro Thr Pro Asp Pro Glu Gly Asp Arg Val Arg Ala Glu Met
               500                 505                 510
   Pro Asn Gln Leu Arg Lys Gln Leu Glu Ala Ile Ile Ala Thr Asp Pro
           515                 520                 525
   Leu Asn Pro Leu Thr Ala Glu Asp Lys Glu Leu Leu Trp His Phe Arg
       530                 535                 540
   Tyr Glu Ser Leu Lys His Pro Lys Ala Tyr Pro Lys Leu Phe Ser Ser
   545                 550                 555                 560
   Val Lys Trp Gly Gln Gln Glu Ile Val Ala Lys Thr Tyr Gln Leu Leu
                   565                 570                 575
   Ala Arg Arg Glu Val Trp Asp Gln Ser Ala Leu Asp Val Gly Leu Thr
               580                 585                 590
   Met Gln Leu Leu Asp Cys Asn Phe Ser Asp Glu Asn Val Arg Ala Ile
           595                 600                 605
   Ala Val Gln Lys Leu Glu Ser Leu Glu Asp Asp Asp Val Leu His Tyr
       610                 615                 620
   Leu Leu Gln Leu Val Gln Ala Val Lys Phe Glu Pro Tyr His Asp Ser
   625                 630                 635                 640
   Ala Leu Ala Arg Phe Leu Leu Lys Arg Gly Leu Arg Asn Lys Arg Ile
                   645                 650                 655
   Gly His Phe Leu Phe Trp Phe Leu Arg Ser Glu Ile Ala Gln Ser Arg
               660                 665                 670
   His Tyr Gln Gln Arg Phe Ala Val Ile Leu Glu Ala Tyr Leu Arg Gly
           675                 680                 685
   Cys Gly Thr Ala Met Leu His Asp Phe Thr Gln Gln Val Gln Val Ile
       690                 695                 700
   Glu Met Leu Gln Lys Val Thr Leu Asp Ile Lys Ser Leu Ser Ala Glu
   705                 710                 715                 720
   Lys Tyr Asp Val Ser Ser Gln Val Ile Ser Gln Leu Lys Gln Lys Leu
                   725                 730                 735
   Glu Asn Leu Gln Asn Ser Gln Leu Pro Glu Ser Phe Arg Val Pro Tyr
               740                 745                 750
   Asp Pro Gly Leu Lys Ala Gly Ala Leu Ala Ile Glu Lys Cys Lys Val
           755                 760                 765
   Met Ala Ser Lys Lys Lys Pro Leu Trp Leu Glu Phe Lys Cys Ala Asp
       770                 775                 780
   Pro Thr Ala Leu Ser Asn Glu Thr Ile Gly Ile Ile Phe Lys His Gly
   785                 790                 795                 800
   Asp Asp Leu Arg Gln Asp Met Leu Ile Leu Gln Ile Leu Arg Ile Met
                   805                 810                 815
   Glu Ser Ile Trp Glu Thr Glu Ser Leu Asp Leu Cys Leu Leu Pro Tyr
               820                 825                 830
   Gly Cys Ile Ser Thr Gly Asp Lys Ile Gly Met Ile Glu Ile Val Lys
           835                 840                 845
   Asp Ala Thr Thr Ile Ala Lys Ile Gln Gln Ser Thr Val Gly Asn Thr
       850                 855                 860
   Gly Ala Phe Lys Asp Glu Val Leu Asn His Trp Leu Lys Glu Lys Ser
   865                 870                 875                 880
   Pro Thr Glu Glu Lys Phe Gln Ala Ala Val Glu Arg Phe Val Tyr Ser
                   885                 890                 895
   Cys Ala Gly Tyr Cys Val Ala Thr Phe Val Leu Gly Ile Gly Asp Arg
               900                 905                 910
   His Asn Asp Asn Ile Met Ile Thr Glu Thr Gly Asn Leu Phe His Ile
           915                 920                 925
   Asp Phe Gly His Ile Leu Gly Asn Tyr Lys Ser Phe Leu Gly Ile Asn
       930                 935                 940
   Lys Glu Arg Val Pro Phe Val Leu Thr Pro Asp Phe Leu Phe Val Met
   945                 950                 955                 960
   Gly Thr Ser Gly Lys Lys Thr Ser Pro His Phe Gln Lys Phe Gln Asp
                   965                 970                 975
   Ile Cys Val Lys Ala Tyr Leu Ala Leu Arg His His Thr Asn Leu Leu
               980                 985                 990
   Ile Ile Leu Phe Ser Met Met Leu Met Thr Gly Met Pro Gln Leu Thr
           995                 1000                1005
   Ser Lys Glu Asp Ile Glu Tyr Ile Arg Asp Ala Leu Thr Val Gly Lys
       1010                1015                1020
   Asn Glu Glu Asp Ala Lys Lys Tyr Phe Leu Asp Gln Ile Glu Val Trp
   1025                1030                1035                1040
   Gln Arg Gln Arg Met Asp Cys Ala Val
   1045
(2) INFORMATION ZU SEQ ID NO: 3:
   (i) SEQUENZ CHARAKTERISTIKA:
      (A) LÄNGE: 4137 Basenpaare
      (B) ART: Nukleinsäure
      (C) STRANGFORM: beides
      (D) TOPOLOGIE: linear
   (ii) ART DES MOLEKÜLS: cDNS
   (vi) URSPRÜNGLICHE HERKUNFT:
      (A) ORGANISMUS: Homo sapiens
   (ix) MERKMALE:
      (A) NAME/SCHLÜSSEL: CDS
      (B) LAGE: 423..3572
   (xi) SEQUENZBESCHREIBUNG: SEQ ID NO: 3:
   GAATTCGGCA CGAGCACTTC CTTCTCGGCT AGATTATCTG AAACTGTTGT CGGTTCTTGA      60
   GATGATACTA CCACCGAATG TCTGTGTTTC ATTGTCTAGT CCAACCTGTA TTGTGGATAT     120
   CTACAACGTT CCGGCAATAG TTTTGCAGGT GCATCACATT TTTGTTTTTG TTTTGGGAGG     180
   AAAAGGGAGG GCACGGCAGC CAGGCTTCAT ATTCCTACAA GTGCATGCTT CAAGATTACT     240
   GTACTTACAG TGTTTCCAAC ATCTTCTCAT AAAAGGGGAA AGCTTCATAG CCTCAACCAT     300
   GAAGGAAACC AGTCGCATAG GGCATGGAGC TGGAGAACTA TAAACAGCCC GTGGTGCTGA     360
   GAGAGGACAA CTGCCGAAGG CGCCGGAGGA TGAAGCCGCG CAGTGCTGCC AGCCTGTCCT     420
   CC ATG GAG CTC ATC CCC ATC GAG TTC GTG CTG CCC ACC AGC CAG CGC        467
   AAA TGC AAG AGC CCC GAA ACG GCG CTG CTG CAC GTG GCC GGC CAC GGC       515
   AAC GTG GAG CAG ATG AAG GCC CAG GTG TGG CTG CGA GCG CTG GAG ACC       563
   AGC GTG GCG GCG GAC TTC TAC CAC CGG CTG GGA CCG CAT CAC TTC CTC       611
   CTG CTC TAT CAG AAG AAG GGG CAG TGG TAC GAG ATC TAC GAC AAG TAC       659
   CAG GTG GTG CAG ACT CTG GAC TGC CTG CGC TAC TGG AAG GCC ACG CAC       707
   CGG AGC CCG GGC CAG ATC CAC CTG GTG CAG CGG CAC CCG CCC TCC GAG       755
   GAG TCC CAA GCC TTC CAG CGG CAG CTC ACG GCG CTG ATT GGC TAT GAC       803
   GTC ACT GAC GTC AGC AAC GTG CAC GAC GAT GAG CTG GAG TTC ACG CGC      851
   CGT GGC TTG GTG ACC CCG CGC ATG GCG GAG GTG GCC AGC CGC GAC CCC      899
   AAG CTC TAC GCC ATG CAC CCG TGG GTG ACG TCC AAG CCC CTC CCG GAG      947
   TAC CTG TGG AAG AAG ATT GCC AAC AAC TGC ATC TTC ATC GTC ATT CAC      995
   CGC AGC ACC ACC AGC CAG ACC ATT AAG GTC TCA CCC GAC GAC ACC CCC     1043
   GGC GCC ATC CTG CAG AGC TTC TTC ACC AAG ATG GCC AAG AAG AAA TCT     1091
   CTG ATG GAT ATT CCC GAA AGC CAA AGC GAA CAG GAT TTT GTG CTG CGC     1139
   GTC TGT GGC CGG GAT GAG TAC CTG GTG GGC GAA ACG CCC ATC AAA AAC     1187
   TTC CAG TGG GTG AGG CAC TGC CTC AAG AAC GGA GAA GAG ATT CAC GTG     1235
   GTA CTG GAC ACG CCT CCA GAC CCG GCC CTA GAC GAG GTG AGG AAG GAA     1283
   GAG TGG CCG CTG GTG GAC GAC TGC ACG GGA GTC ACC GGC TAC CAT GAG     1331
   CAG CTT ACC ATC CAC GGC AAG GAC CAC GAG AGT GTG TTC ACC GTG TCC     1379
   CTG TGG GAC TGC GAC CGC AAG TTC AGG GTC AAG ATC AGA GGC ATT GAT     1427
   ATC CCC GTC CTG CCT CGG AAC ACC GAC CTC ACA GTT TTT GTA GAG GCA     1475
   AAC ATC CAG CAT GGG CAA CAA GTC CTT TGC CAA AGG AGA ACC AGC CCC     1523
   AAA CCC TTC ACA GAG GAG GTG CTG TGG AAT GTG TGG CTT GAG TTC AGT     1571
   ATC AAA ATC AAA GAC TTG CCC AAA GGG GCT CTA CTG AAC CTC CAG ATC     1619
   TAC TGC GGT AAA GCT CCA GCA CTG TCC AGC AAG GCC TCT GCA GAG TCC     1667
   CCC AGT TCT GAG TCC AAG GGC AAA GTT CGG CTT CTC TAT TAT GTG AAC        1715
   CTG CTG CTG ATA GAC CAC CGT TTC CTC CTG CGC CGT GGA GAA TAC GTC        1763
   CTC CAC ATG TGG CAG ATA TCT GGG AAG GGA GAA GAC CAA GGA AGC TTC        1811
   AAT GCT GAC AAA CTC ACG TCT GCA ACT AAC CCA GAC AAG GAG AAC TCA        1859
   ATG TCC ATC TCC ATT CTT CTG GAC AAT TAC TGC CAC CCG ATA GCC CTG        1907
   CCT AAG CAT CAG CCC ACC CCT GAC CCG GAA GGG GAC CGG GTT CGA GCA        1955
   GAA ATG CCC AAC CAG CTT CGC AAG CAA TTG GAG GCG ATC ATA GCC ACT        2003
   GAT CCA CTT AAC CCT CTC ACA GCA GAG GAC AAA GAA TTG CTC TGG CAT        2051
   TTT AGA TAC GAA AGC CTT AAG CAC CCA AAA GCA TAT CCT AAG CTA TTT        2099
   AGT TCA GTG AAA TGG GGA CAG CAA GAA ATT GTG GCC AAA ACA TAC CAA        2147
   TTG TTG GCC AGA AGG GAA GTC TGG GAT CAA AGT GCT TTG GAT GTT GGG        2195
   TTA ACA ATG CAG CTC CTG GAC TGC AAC TTC TCA GAT GAA AAT GTA AGA        2243
   GCC ATT GCA GTT CAG AAA CTG GAG AGC TTG GAG GAC GAT GAT GTT CTG        2291
   CAT TAC CTT CTA CAA TTG GTC CAG GCT GTG AAA TTT GAA CCA TAC CAT        2339
   GAT AGC GCC CTT GCC AGA TTT CTG CTG AAG CGT GGT TTA AGA AAC AAA        2387
   AGA ATT GGT CAC TTT TTG TTT TGG TTC TTG AGA AGT GAG ATA GCC CAG        2435
   TCC AGA CAC TAT CAG CAG AGG TTC GCT GTG ATT CTG GAA GCC TAT CTG        2483
   AGG GGC TGT GGC ACA GCC ATG CTG CAC GAC TTT ACC CAA CAA GTC CAA      2531
   GTA ATC GAG ATG TTA CAA AAA GTC ACC CTT GAT ATT AAA TCG CTC TCT      2579
   GCT GAA AAG TAT GAC GTC AGT TCC CAA GTT ATT TCA CAA CTT AAA CAA      2627
   AAG CTT GAA AAC CTG CAG AAT TCT CAA CTC CCC GAA AGC TTT AGA GTT      2675
   CCA TAT GAT CCT GGA CTG AAA GCA GGA GCG CTG GCA ATT GAA AAA TGT      2723
   AAA GTA ATG GCC TCC AAG AAA AAA CCA CTA TGG CTT GAG TTT AAA TGT      2771
   GCC GAT CCT ACA GCC CTA TCA AAT GAA ACA ATT GGA ATT ATC TTT AAA      2819
   CAT GGT GAT GAT CTG CGC CAA GAC ATG CTT ATT TTA CAG ATT CTA CGA      2867
   ATC ATG GAG TCT ATT TGG GAG ACT GAA TCT TTG GAT CTA TGC CTC CTG      2915
   CCA TAT GGT TGC ATT TCA ACT GGT GAC AAA ATA GGA ATG ATC GAG ATT      2963
   GTG AAA GAC GCC ACG ACA ATT GCC AAA ATT CAG CAA AGC ACA GTG GGC      3011
   AAC ACG GGA GCA TTT AAA GAT GAA GTC CTG AAT CAC TGG CTC AAA GAA      3059
   AAA TCC CCT ACT GAA GAA AAG TTT CAG GCA GCA GTG GAG AGA TTT GTT      3107
   TAT TCC TGT GCA GGC TAC TGT GTG GCA ACC TTT GTT CTT GGA ATA GGC      3155
   GAC AGA CAC AAT GAC AAT ATT ATG ATC ACC GAG ACA GGA AAC CTA TTT      3203
   CAT ATT GAC TTC GGG CAC ATT CTT GGG AAT TAC AAA AGT TTC CTG GGC      3251
   ATT AAT AAA GAG AGA GTG CCA TTT GTG CTA ACC CCT GAC TTC CTC TTT      3299
   GTG ATG GGA ACT TCT GGA AAG AAG ACA AGC CCA CAC TTC CAG AAA TTT      3347
   CAG GAC ATC TGT GTT AAG GCT TAT CTA GCC CTT CGT CAT CAC ACA AAC      3395
   CTA CTG ATC ATC CTG TTC TCC ATG ATG CTG ATG ACA GGA ATG CCC CAG      3443
   TTA ACA AGC AAA GAA GAC ATT GAA TAT ATC CGG GAT GCC CTC ACA GTG      3491
   GGG AAA AAT GAG GAG GAT GCT AAA AAG TAT TTT CTT GAT CAG ATC GAA      3539
   GTT TGG CAG AGA CAA AGG ATG GAC TGT GCA GTT TAATTGGTTT CTACATCTTG    3592
   TTCTTGGCAT CAAACAAGGA GAGAAACATT CAGCCTAATA CTTTAGGCTA GAATCAAAAA    3652
   CAAGTTAGTG TTCTATGGTT TAAATTAGCA TAGCAATCAT CGAACTTGGA TTTCAAATGC    3712
   AATAGACATT GTGAAAGCTG GCATTTCAGA AGTATAGCTC TTTTCCTACC TGAACTCTTC    3772
   CCTGGAGAAA AGATGTTGGC ATTGCTGATT GTTTGGTTAA GCAATGTCCA GTGCTAGGAT    3832
   TATTTGCAGG TTTGGTTTTT TCTCATTTGT CTGTGGCATT GGAGAATATT CTCGGTTTAA    3892
   ACAGACTAAT GACTTCCTTA TTGTCCCTGA TATTTTGACT ATCTTACTAT TGAGTGCTTC    3952
   TGGAAATTCT TTGGAATAAT TGATGACATC TATTTTCATC TGGGTTTAGT CTCAATTTTG    4012
   GTTATCTTTG TGTTCCTCAA GCTCTTTAAA GAAAAAGATG TAATCGTTGT AACCTTTGTC    4072
   TCATTCCTTA AATGATGCTT CCAAACATCT CCTTAGTGTC TGCAGGTGTT AGTGGTGTGC    4132
   TAAAA                                                                4137
(2) INFORMATION ZU SEQ ID NO: 4:
   (i) SEQUENZ CHARAKTERISTIKA:
      (A) LÄNGE: 1050 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 4:

## Beschreibung

Die vorliegende Erfindung betrifft eine neue Phosphatidylinositol-3-Kinase (PI3Kγ), eine dafür kodierende Nucleinsäure, einen gegen das Protein gerichteten Antikörper sowie die diagnostische und therapeutische Anwendung des Proteins, der Nucleinsäure und des Antikörpers.

Phosphatidylinositol-Kinasen gehören neben spezifischen Phospholipasen zu einer Enzymgruppe, welche die Bildung von intrazellulären Botensubstanzen aus dem Membranlipid Phosphatidylinositol (PI) katalysiert. Die Aktivität dieser Enzyme wird durch extrazelluläre Effektoren wie Hormone, Wachstumsfaktoren und Neurotransmitter reguliert. Man nimmt an, daß die PI abhängigen Botenstoffe an der Regulation wichtiger Zellfunktionen, wie etwa der Zellproliferation, der Sekretion von Zellinhaltsstoffen, endozytotischen Vorgängen, der gerichteten Bewegung bestimmter Zellen, gesteuerten Veränderungen des Zytoskeletts u.a. Prozessen beteiligt sind. Der physiologischen Bedeutung der PI-Kinasen und Phospholipasen entsprechend korrelieren eine Reihe von Krankheitsbildern mit Veränderungen der Funktionen dieser Enzyme.

Aus verschiedenen experimentellen Ergebnissen kann man schließen, daß das Produkt der durch die PI3-Kinase katalysierte Reaktion PI,-3,4,5-triphosphat eine wesentliche Rolle bei der Regulation der im folgenden genannten physiologischen Zellfunktionen spielt:
- Regulation der Zellprofileration- und differenzierung durch PI 3-Kinase
   Mitogene, wie Wachstumsfaktoren und Zytokine führen im allgemeinen zu einer Stimulation der PI 3-Kinaseeaktivität in teilungsfähigen Zellen. Auch die onkogene Transformation von Zellen wird vielfach von einer Erhöhung der meßbaren PI 3-Kinase-Aktivät begleitet (Varticovski et al., Biochim.Biophys.Acta 1226, 1-11 (1994), Berggren et al., Cancer Research 53, 4297-4302 (1993), Soldi et al., Oncogene 9, 2253-2260 (1994)). Inhibitoren der PI 3-Kinase sind in der Lage, das durch PDGF stimulierte Wachstum von normalen Bindegewebszellen oder glatten Muskelzellen und die Proliferation von src-supratransformierten Fibroblasten (Krebszellen) zu hemmen (Berggren et al., Cancer Research 53, 4297-4302 (1993), Vlahos et al., J.Biol.Chem. 269, 5241-5248 (1994)). Berggren et al., vermuten, daß die tumorstatische Wirkung von Ether-Lipidanalogen wesentlich auf deren hemmende Wirkung auf die PI 3-Kinase beruht.
   Die Differenzierung der Nervenzellinie PC12 wird durch den Hemmstoff der PI 3-Kinase Wortmannin unterdrückt (Kimurea et al., J.Biol.Chem. 269, 18961-18967 (1994)). Diese Befunde wie auch eine klinische Studie, bei der ein selektiver Verlust von PI 3-Kinase-Aktivät im Hirn von Alzheimerpatienten nachgewiesen wurde (Bothmer et al., Dementia 5, 6-11 (1994)), deuten auf eine wesentliche Funktion des Enzyms bei der Ausbildung und Erhaltung von Nervengewebe hin.
- Regulation von Zytoskelett-abhängigen Prozesen durch PI 3-Kinase
   Mikroskopisch sichtbare Veränderungen von Zellen verlaufen häufig unter Beteiligung des Zytoskeletts. Eine Reihe von Ergebnissen zeigen, daß solche Vorgänge zumindest zum Teil durch PI 3-Kinase und deren enzymatische Produkte (PI3,4,5,P₃, PI3,4P₂ und PI3P) gesteuert werden. So kann das durch Insulin oder PDGF induzierte "membrane ruffling" epidermaler Zellen durch den PI 3-Kinase-Inhibitor Wortmannin unterdrückt werden (Kotani et al., EMBO J. 13, 2313-2321 (1994), Wennström et al., Curr.Biol. 4, 385-393 (1994)).
   Basophile Leukozyten sind in der Lage, Histamin - einen Mediator von Entzündungen und allergischen Erscheinungen - auszuschütten. Dieser Sekretionsprozeß verläuft unter Beteiligung des Zytoskeletts der Zellen. Yano et al., J. Biol.Chem. 268, 25846-25856 (1993) konnten nachweisen, daß die durch Antikörper induzierte Histaminsekretion wiederum durch den PI 3-Kinase-Inhibitor Wortmannin gehemmt werden kann, also offenbar durch 3-phosphorylierte Phosphoinositide gesteuert wird.
- Beteiligung der PI 3-Kinase an intrazellulären Transportprozessen
   Untersuchungen von Hefemutanten zeigen, daß eine Form der PI 3-Kinase (Vps 34) in diesen Organismen an der selekiven Verteilung von Proteinen in Richtung der Hefevacuole beteiligt ist (Schu et al., Science 260, 88-91 (1993)). Ähnliche Mechanismen liegen möglicherweise der durch Insulin stimulierten Translokation von Glukosetransportprotein (GLUT 4) aus dem Zellinneren zur Plasmamembran zugrunde (Kanai et al., Biochem.Biophys.Res.Commun. 195, 762-768 (1993)). Auch dieser in verschiedenen Organen wichtige Vorgang wird durch Wortmannin inhibiert und läuft offenbar unter Beteiligung von PI 3-Kinase ab.
- Hemmung der O₂⁻Produktion in neutrophilen Granulozyten
   Mit dem Ziel phagozytierte Fremdzellen zu vernichten produzieren Granulozyten Superoxidanionen (O₂⁻). Dieser Vorgang wird durch das Chemoattraktanz fMLP stimuliert. Die Blockade der fMLP-induzierten O₂⁻-Bildung durch Wortmannin deutet auf die regulatorische Funktion einer PI 3-Kinase-Spezies an diesem für die Immunantwort des Körpers wichtigen Vorgang hin.

Die aufgeführten Ergebnisse weisen auf die zentrale Bedeutung der PI 3-Kinase und 3-phosphorylierter Inositollipide bei der Regulation wichtiger Zellfunktionen hin. Zweifellos liegen wichtigen Krankheitsbildern erworbene oder ererbte Defekte der genannten Zellfunktionen zugrunde. Als Beispiele seien genannt: Krebs, Arteriosklerose, Immunopathien, Hautkrankheiten (wie Psoriasis), degenerative Erkrankungen des Nervensystems.

Da die PI 3-Kinase ein essentielles Element bei der Regulation der aufgeführten Zellfunktionen ist, lassen sich mit hoher Wahrscheinlichkeit einige der Krankheitsbilder auf Fehlfunktionen von PI 3-Kinase-Spezies zurückführen. Die klinische Studie zur PI 3-Kinase im Hirn von Alzheimerpatienten, die Befunde zur Rolle der PI 3-Kinase bei der Bildung des Allergieinduktors Histamin und auch die kanzerostatische Wirkung der PI 3-Kinase inhibierenden Etherlipide deuten in dieser Richtung.

Es ist ein zentrales Anliegen der Zellbiologie, die Mechanismen der intrazellulären Signalübertragung und die beteiligten Botenstoffe aufzudecken. Diese Untersuchungen dienen letztlich dem Ziel, Zellfunktionen im medizinischen Sinne selektiv zu beeinflussen.

Die vorliegende Anmeldung beschreibt die Identifizierung, Klonierung, Expression und Charakterisierung einer neuen Spezies PI 3-Kinase. Diese neue Spezies wird durch G-Protein-Untereinheiten aktiviert und unterscheidet sich damit von den bisher aus Säugetierzellen klonierten Spezies PI3Kα (Hiles et al., Cell 70 (1992) 419-429) und PI3Kβ (Hu et al., J. Mol.Cell.Biol. 13, (1993), 7677-7688) sowie der PI3K-Vps34 aus Hefe (Schu et al., supra und Herman et al., Cell 64 (1991), 425-438) durch den Regulationsmechanismus. Die funktionellen Unterschiede zwischen den bekannten Enzymen PI3Kα und PI3Kβ einerseits sowie dem erfindungsgemäßen Enzym PI3Kγ andererseits finden ihre Entsprechung in Sequenzunterschieden in den regulatorisch wichtigen Domänen des Enzyms.

Ein Gegenstand der vorliegenden Erfindung ist somit ein Protein mit Phosphatidylinositol-3-Kinase-Aktivität, welches dadurch gekennzeichnet ist, daß es
a) die in SEQ ID NO. 2 dargestellte Aminosäuresequenz,
b) die in SEQ ID NO. 4 dargestellte Aminosäuresequenz oder
c) Varianten der Sequenz aus (a) umfaßt.

Vorzugsweise ist die erfindungsgemäße PI3Kγ ein aus dem Menschen erhältliches Protein, d.h. es ist das in SEQ ID NO. 1 und NO. 2 gezeigte Protein, das in SEQ ID NO. 3 und NO. 4 gezeigte Protein oder eine natürlich vorkommende humane Variante davon.

Ein Gegenstand der Erfindung ist auch ein neues Protein, das Teile der in SEQ ID NO. 1 und 2 bzw. SEQ ID NO. 3 und 4 dargestellten Aminosäuresequenz umfaßt. Vorzugsweise betrifft die Erfindung eine PI3K, welches die in SEQ ID NO. 1 und 2 dargestellte Aminosäuresequenz oder die in SEQ ID N0. 3 und 4 dargestellte Aminosäuresequenz umfaßt, es kann jedoch auch Varianten dieser Sequenz enthalten. Unter dem Begriff "Variante" im Sinne der vorliegenden Erfindung sind Sequenzen zu verstehen, die durch Substitution, Deletion oder/und Insertion einzelner Aminosäuren oder kurzer Aminosäureabschnitte sich von der in SEQ ID NO. 1 und 2 bzw. der in SEQ ID NO. 3 und 4 dargestellten Aminosäuresequenz unterscheiden.

Unter dem Begriff "Variante" fallen sowohl natürlich vorkommende allelische Variationen der PI3Kγ sowie durch rekombinante DNA-Technologie (insbesondere durch in vitro-Mutagenese mit Hilfe von chemisch synthetisierten Oligonukleotiden) erzeugte Proteine, die hinsichtlich ihrer biologischen oder/und immunologischen Aktivität dem in SEQ ID NO. 1 und 2 dargestellten Protein bzw. dem in SEQ ID NO. 3 und 4 dargestellten Protein im wesentlichen entsprechen.

Erfindungsgemäße Proteine zeichnen sich vorzugsweise dadurch aus, daß sie auf Aminosäureebene eine Homologie von mindestens 80 %, besonders bevorzugt 90 % und am meisten bevorzugt 95 % gegenüber der in SEQ ID NO. 1 und 2 dargestellten Aminosäuresequenz oder der in SEQ ID NO. 3 und 4 dargestellten Aminosäuresequenzen aufweisen.

Die in SEQ ID NO. 1 und 2 gezeigte Aminosäuresequenz stellt eine gesamte PI3Kγ dar. Dieses Protein weist 1049 Aminosäuren auf und besitzt eine Molekularmasse von ca. 120 kDa. Die in SEQ ID N0. 3 und 4 gezeigte Aminosäuresequenz stellte eine PI3Kγ mit 1050 Aminosäuren dar.

Die Aminosäuresequenz von PI3Kγ zeigt eine Homologie von 19 bis 39 % zu den den Sequenzen anderer bekannter PI3K, wie etwa humane PI3Kα (36 % Homologie), humane PI3Kβ (33,5 % Homologie) und PI3K-Vps34 aus Hefe (27,7 %). Die am höchsten konservierte Region in dieser Gruppe von Enzymen sind die 400 C-terminalen Aminosäurereste, welche vermutlich die Kinasedomäne enthalten. Es gibt keine signifikante Homologie zwischen PI3Kγ und den anderen Enzymen in der Amino-terminalen Region, von der man weiß, daß sie für die Bindung von PI3Kα und β-Enzymuntereinheiten an die regulatorischen und adaptorischen Untereinheiten p85α und p85β verantwortlich sind (Dhand et al., EMBO J. 13 (1994), 511-521).

PI3Kγ kann in der Zelle durch Immunpräzipation und Western-Blot-Analyse von U937 und K562 Zellen als 110 kDa Protein unter Verwendung von Antipeptid-Antiseren nachgewiesen werden. Eine Northern-Blot-Analyse zeigte eine 5,3 kb lange mRNA in mehreren unterschiedlichen Gewebearten.

Ein weiterer Gegenstand der vorliegenden Erfindung ist eine Nucleinsäure, die für eine erfindungsgemäße Phospatidylinositol-3-Kinase oder Teile davon kodiert. Diese Nucleinsäure kann z.B. genomische DNA, cDNA oder RNA sein. Vorzugsweise handelt es sich um ein rekombinantes DNA-Molekül.

Weiterhin ein Gegenstand der Erfindung ist eine Nucleinsäure, welche
a) die in SEQ ID NO. 1 dargestellte kodierende Sequenz,
b) die in SEQ ID NO. 3 dargestellte Protein-kodierende Sequenz,
c) eine der Sequenz aus (a) oder (b) im Rahmen der Degneration des genetischen Codes entsprechende Nucleinsäuresequenz oder
d) eine mit den Sequenzen aus (a), (b) und/und (c) unter stringenten Hybridisierungsbedingungen hybridisierende Sequenz enthält.

Unter stringenten Hybridisierungsbedingungen im Sinne der vorliegenden Erfindung versteht man, daß eine Hybridisierung auch nach Waschen bei 55 °C, vorzugsweise bei 62 °C, besonders bevorzugt bei 68 °C in einem wäßrigen Niedrigsalz-Puffer (z.B. 0,2 x SSC, 0,1 % SDS) noch auftritt (siehe auch Sambrook et al. (1989) Molecular Cloning, A Laboratory Manual).

Die Erfindung betrifft auch Nucleinsäuren, die einen mindestens 20 Nucleotide langen Abschnitt der in SEQ ID NO. 1 oder SEQ ID NO. 3 dargestellten Sequenz enthalten. Vorzugsweise besitzt dieser Abschnitt eine für die PI3Kγ-spezifische Nucleotidsequenz. Diese Nucleinsäuren eignen sich insbesondere zur Herstellung von therapeutisch einsetzbaren Antisense-Nucleinsäuren, die vorzugsweise bis zu 50 Nucleotide lang sind.

Noch ein weiterer Gegenstand der Erfindung ist ein Vektor, der mindestens eine Kopie einer erfindungsgemäßen Nucleinsäure oder einen Teil davon enthält. Der Vektor kann in Eukaryonten oder Prokaryonten replizierbar sein. Er kann ein in das Genom der Wirtszelle integrierbarer Vektor, z.B. Bakteriophage Lambda, oder ein Vektor sein, der extrachromosomal vorliegt (z.B. ein Plasmid). Der erfindungsgemäße Vektor kann durch Subklonierung der PI3Kγ DNA in einen Basisvektor erhalten werden. Derartige Basisvektoren, insbesondere Vektoren mit den zur Proteinexpression erforderlichen Elementen sind einem Fachmann geläufig.

Bei Klonierung einer für PI3Kγ kodierenden Nucleinsäure kann ein Expressionsvektor hergestellt werden, der in einer geeigneten Wirtszelle zur Expression gebracht wird, wobei das erfindungsgemäße Protein entsteht. Bevorzugte Wirtszellen sind Mikroorganismen wie E. coli oder Hefe, aber auch höhere Zellen (z.B. Säuger- oder Insektenzellen). Bevorzugte Expressionsvektoren sind z.B. Plasmide, Bakteriophage Lambda für Prokaryonten, Hefevektoren oder virale Vektoren für höhere Zellen (z.B. SV40, Vaccinia, Baculoviren). Bezüglich der Expression einer für PI3Kγ kodierten Nucleinsäure soll insbesondere auf die bei Sambrook et al. (1989), supra genannten Methoden verwiesen werden.

Ein spezifisches Beispiel für ein zur Expression von PI3Kγ geeignetes System ist die Expression als GST-Fusionsprotein in Sf9-Insektenzellen unter Verwendung von Baculovektoren gemäß der bei Davis et al. (Biotechnology 11 (1993), 933-936) beschriebenen Methode.

Ein weiterer Gegenstand der vorliegenden Erfindung ist eine Zelle, die mit einer erfindungsgemäßen Nucleinsäure oder einem erfindungsgemäßen Vektor transformiert ist. Die Zelle kann sowohl eine eukaryontische als auch eine prokaryontische Zelle sein. Verfahren zur Transformation von Zellen mit Nucleinsäuren sind allgemeiner Stand der Technik und brauchen daher nicht näher erläutert werden.

Ebenfalls ein Gegenstand der vorliegenden Erfindung ist die Verwendung des PI3Kγ Proteins oder Fragmenten dieses Proteins als Immunogen zur Herstellung von Antikörpern. Die Herstellung von Antikörpern kann dabei auf übliche Weise durch Immunosierung von Versuchstieren mit dem vollständigen PI3Kγ Protein oder Fragmenten davon und anschließende Gewinnung der resultierenden polyklonalen Antiseren erfolgen. Nach der Methode von Köhler und Milstein oder deren Weiterentwicklungen können aus den Antikörper-produzierenden Zellen der Versuchstiere auf bekannte Weise durch Zellfusion monoklonale Antikörper erhalten werden. Ebenso können humane monoklonale Antikörper hergestellt werden.

Ein weiterer Gegenstand der vorliegenden Erfindung ist somit ein Antikörper gegen ein Protein mit Phosphatidylinositol-3-Kinase-Aktivität, der für Phosphatidylinositol-3-Kinase-γ spezifisch und keine Kreuzreaktion mit anderen Phosphatidylinositol-3-Kinasen zeigt. Ein solcher Antikörper ist beispielsweise durch Verwendung einer PI3Kγ-spezifischen Peptidsequenz als Immunogen erhältlich, z.B. einer Peptidsequenz, die aus den Aminosäuren 741 bis 755 der in SEQ ID NO. 1 und 2 darstellten Aminosäuresequenz bzw. den Aminosäuren 742 bis 756 der in SEQ ID N0. 3 und 4 dargestellten Aminosäuresequenz entspricht.

Die Bereitstellung der PI3 Kinase γ, einer dafür kodierenden Nucleinsäure und eines dagegen gerichteten Antikörpers schafft die Voraussetzung für eine gezielte Suche nach Effektoren dieses Proteins. Angriffspunkt für diese Substanzen sollten die regulatorischen Domänen des Enzyms sein, die sich im Bereich der Aminosäurereste 1 bis 700 der in SEQ ID NO. 1 und 2 bzw. SEQ ID N0. 3 und 4 dargestellten Aminosäuresequenzen befinden. Stoffe, die über diese Region des Proteins inhibitorisch oder aktivierend auf die Aktivität wirken, sind in der Lage, durch PI3Kγ-gesteuerten Zellfunktionen selektiv zu beeinflussen. Daher können sie bei der Therapie entsprechender Krankheitsbilder eingesetzt werden. Bei Krankheitsbildern, die auf einen Ausfall der PI3Kγ zurückzuführen sind, könnte eine gentherapeutische Behandlung erfolgen, welche die Übertragung einer für PI3Kγ-kodierenden Nucleinsäure, ggf. zusammen mit einer Nucleinsäure, welche für die aktivierenden G-Proteine kodiert, mittels Vektoren, z.B. viralen Vektoren in das entsprechende Zielgewebe umfaßt.

Die vorgestellten Ergebnisse schaffen überdies die Voraussetzungen für eine gezielte Diagnostik von Krankheiten, die mit Veränderungen der PI3Kγ-Aktivität ursächlich verbunden sind. Diese Untersuchungen können mit Hilfe spezifischer Nucleinsäuresonden zum Nachweis auf DNA-Ebene, d.h. auf Gen- bzw. Transkriptebene oder mit Hilfe von Antikörpern gegen PI3Kγ zum Nachweis auf Proteinebene durchgeführt werden.

Die vorliegende Erfindung betrifft somit auch eine pharmazeutische Zusammensetzung, die als aktiven Bestandteil ein PI3Kγ-Protein, einen dagegen gerichteten Antikörper oder einen dafür kodierende Nucleinsäure, ggf. zusammen mit pharmazeutisch üblichen Hilfs-, Träger-, Füll- und Verdünnungsmitteln umfaßt.

Die erfindungsgemäße pharmazeutische Zusammensetzung kann insbesondere zur Beeinflussung der Zellproliferation, der Rezeptor-vermittelten Signalübertragung, der Struktur der Zellmembran, der Ausschüttung von Histaminen, der Differenzierung von Nervenzellen, dem Glucosetransport und der Antilipolyse verwendet werden. Weiterhin ist eine Verwendung im Zusammenhang mit der Therapie der Alzheimerschen Krankheit möglich.

Die Erfindung wird durch die folgenden Beispiele, Abbildungen und Sequenzprotokolle näher erläutert. Es zeigen:
- Abb. 1: den Nachweis von PI3Kγ in humanen Blutzellen über spezifische Antikörper,
- Abb. 2a: die rekombinante Expression von PI3Kγ in Insektenzellen,
- Abb. 2b: den Nachweis der enzymatischen Aktivität von gereinigter rekombinanter PI3Kγ,
- Abb. 3: die fehlende Wechselwirkung von PI3Kγ mit den Proteinen p85α und p85β,
- SEQ ID NO. 1: eine Nucleinsäuresequenz, die eine für PI3Kγ-kodierende genetische Information enthält und
- SEQ ID NO. 2: die Aminosäuresequenz einer PI3Kγ.
- SEQ ID NO. 3: eine Nucleinsäuresequenz, die eine für eine weitere PI3Kγ-kodierende genetische Information und
- SEQ ID N0. 4: die Aminosäuresequenz einer weiteren PI3Kγ.

### Beispiel 1

### Isolierung der PI3Kγ cDNA

Zur Isolierung von cDNA Sequenzen, die für neue PI3K kodieren, wurde eine humane Knochenmark-cDNA-Bank unter Verwendung der Polymerase-Kettenreaktion (PCR) durchmustert. Hierbei wurden degenerierte Oligonucleotidprimer entsprechend den Aminosäuresequenzen KNGDDLR und HIDFG verwendet. Es wurde ein 402 bp langes DNA Fragment erhalten. Dieses Fragment wurde subkloniert und sequenziert.

Unter Verwendung dieses PCR-Fragments als Sonde wurden mehrere überlappende Klone aus einer humanen U937 cDNA-Bank isoliert. Der größte Klon enthielt die in SEQ ID NO. 1 dargestellte Nucleinsäuresequenz mit einem offenen Leserahmen, der für ein Protein mit 1049 Aminosäuren kodiert (SEQ ID NO. 2). Dieses als PI3Kγ bezeichnete Protein hat eine Molekularmasse von ungefähr 120 kDa.

Eine weitere PI3Kγ-Sequenz, die aus einer cDNA-Bank erhalten wurde, ist in SEQ ID NO. 3 dargestellt und kodiert für ein Protein mit 1050 Aminosäuren (SEQ ID N0. 4).

### Beispiel 2

### Nachweis von PI3Kγ auf Protein- und Transkriptebene

Es wurde ein polyklonales Kaninchen-Antiserum gegen PI3Kγ durch Immunisierung mit einem 15 Aminosäuren langen Peptid der Sequenz NSQLPESFRVPYDPG (entsprechend den Aminosäuren 741 bis 755 in SEQ ID NO. 2 bzw. den Aminosäuren 742 bis 756 in SEQ ID N0. 4) hergestellt. Das Serum wurde durch Protein A Chromatographie und Affinitätschromatographie unter Verwendung des an Actigel (Sterogene) gekoppelten Peptidantigens gereinigt.

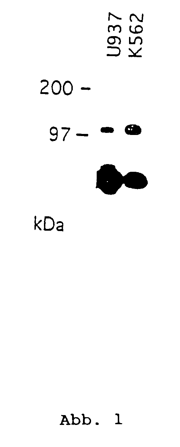

PI3Kγ kann durch Immunpräzipitation und Western-Blot-Analyse von U937 und K562 Zellen als 110kDa Protein unter Verwendung dieses Antiserums nachgewiesen werden (Abb. 1). Die Immunpräzipitation und der Western-Blot wurden nach der Methode von Hiles et al. (Cell 70 (1992) 419-429) durchgeführt. Als sekundärer Antikörper wurde ein Konjugat aus Meerrettich-Peroxidase und Anti-Kaninchen-Antiserum (Sigma, 1:2000 Verdünnung) verwendet. Die gebundene Peroxidase wurde durch Chemilumineszenz sichtbar gemacht.

Eine Northern-Blot-Analyse von menschlichem Gewebe aus Pankreas, Niere, Skelettmuskel, Leber, Lunge, Placenta, Gehirn und Herz zeigte jeweils unterschiedliche Konzentrationen einer 5,3 kb-langen mRNA.

### Beispiel 3

### Rekombinante Expression von PI3Kγ

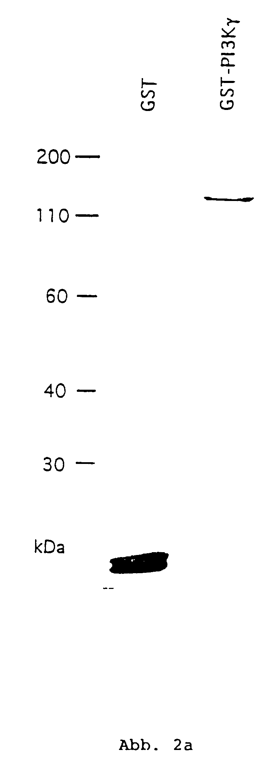

Die für PI3Kγ-kodierende DNA wurde in den Vektor pAcG2T (Davis et al., Biotechnology 11 (1993), 933-936) kloniert. Der resultierende rekombinante Vektor pAcG2T-PI3Kγ enthielt die PI3Kγ-cDNA ab Codon 4 in Fusion mit dem Glutathion S Transferase (GST) Gen. Sf9-Zellen wurden mit pAcG2T-PI3Kγ und linearisierter Baculovirus-DNA (BaculoGold, Pharmingen) cotransfiziert. Einzelne rekombinante Baculovirus GST-PI3Kγ Plaques wurden gereinigt und amplifiziert. Die Expression und Reinigung des rekombinanten Proteins wurden unter Verwendung von Standardprotokollen (Dhand et al., EMBO J. 13 (1994), 511-521) durchgeführt. Abbildung 2a zeigt die Expression von rekombinantem PI3Kγ GST-Fusionsprotein oder GST alleine nach Fraktionierung auf einen SDS Polyacrylamidgel. Der Nachweis erfolgte durch Anfärbung mit Coomassie blau.

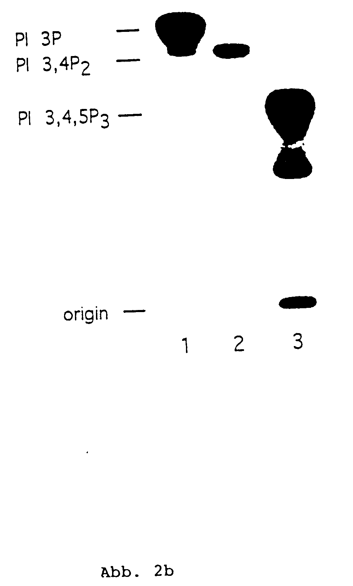

Abbildung 2b zeigt einen Nachweis der enzymatischen Aktivität von gereinigter rekombinanter PI3Kγ. Als Substrate wurden Phosphatidylinositol (PI, Spur 1), Phosphatidylinositol-4-Phosphat (PI4-P, Spur 2) und Phosphatidylinositol-4,5-Diphosphat (PI 4,5-P₂, Spur 3) verwendet. Die Durchführung des Tests wurde im wesentlichen nach der Methode von Stephens et al. (Cell 77 (1994), 83-93) aber ohne Cholat durchgeführt. Es wurden 30 *µ*l sonifizierte Lipidvesikel mit 320 *µ*M Phosphatidylethanolamin, 140 *µ*M Phosphatidylcholin, 300 µM Phosphatidylserin, 30 *µ*M Sphingomyelin und 320 µM Substrat zu 10 µl Enzym (0,1 ng) gegeben und 8 Minuten lang auf Eis inkubiert. Der Test wurde durch Zugabe 10 µl 20 µM ATP mit 10 *µ*Ci γ(³²P)ATP gestartet und 15 Minuten bei Raumtemperatur inkubiert. Die extrahierten Lipide wurden aufgetrennt und sichtbar gemacht. Die Identität der 3-phosphorylierten Phosphoinositide wurde durch Anionenaustauscher-HPLC nach Deacylierung der Lipide bestätigt (Auger et al., Cell 57 (1989), 167-175).

Abbildung 2b zeigt, daß die Substrate in der D-3 Position des Inositolrings phosphoryliert wurden. Weiterhin wurde gefunden, daß PI3Kγ durch Wortmannin bei nanomolaren Konzentrationen inhibiert wurde.

### Beispiel 4

### Regulation von PI3Kγ

Um eine Wechselwirkung zwischen den regulatorischen und adaptorischen Untereinheiten von p85 (p85α bzw. p85β) mit PI3Kγ nachzuweisen, wurden humane PI3Kγ und Rinder PI3Kα (Hiles et al., supra) nach dem in Beispiel 3 beschriebenen Verfahren als GST-Fusionsproteine entweder alleine oder zusammen mit p85α oder p85β in Sf9-Insektenzellen exprimiert. Nach Lyse und Zentrifugation der Zellen wurde Glutathion-Sepharose zum Überstand gegeben, um die GST-Fusionsproteine zu binden. Die Körner wurden gewaschen und durch SDS-PAGE und Western-Blot analysiert. Der Nachweis von PI3Kγ erfolgte unter Verwendung der in Beispiel 2 beschriebenen polyklonalen Antipeptid-Antikörper. Zum Nachweis von p85α, p85β und Rinder PI3Kα wurden entsprechende Mischungen spezifischer monoklonaler Antikörper (Dhand et al., supra) als primäre Antikörper und Konjugate von Meerrettich-Peroxidase und Anti-Maus-Antikörpern (Dianova, 1:4000 Verdünnung) als sekundäre Antikörper verwendet.

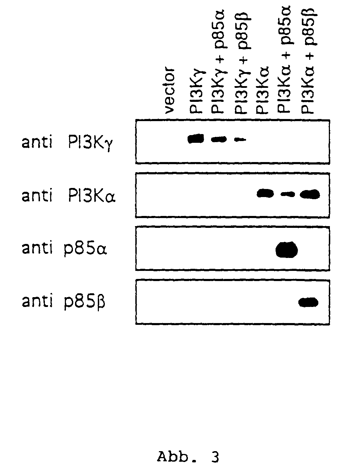

Aus Abbildung 3 ist ersichtlich, daß PI3Kγ im Gegensatz zu PI3Kα nicht an p85α oder p85β Untereinheiten bindet. PI3Kγ unterscheidet sich daher im Regulationsmechanismus von PI3Kγ.

Um eine Wechselwirkung zwischen PI3Kγ und G-Proteinen nachzuweisen, wurden gereinigte rekombinante humane PI3Kγ und Rinder PI3Kα mit der Transducinspezies Gₜβγ inkubiert. Es wurde hierbei eine deutliche Verstärkung der Kinase-Aktivität von PI3Kγ gefunden. Diese Stimulation konnte durch Zusatz von GDP-beladenem Gₜα unterdrückt werden, wodurch die Spezifität der Wechselwirkung bestätigt wurde.

Die enzymatische Aktivität von PI3Kα konnte hingegen nicht durch Zusatz von Gₜß_{γ} stimuliert werden. Eine ähnliche Aktivierung des Enzyms wurde nach Zugabe von Gα in Anwesenheit von 20*µ*M AlCl₃ und 10 mM NaF gefunden. Als aktivierende Spezies wirkt der Komplex Gα-GDP-AIF₄-.

Die Aktivitätsbestimmung erfolgte wie in Beispiel 3 beschrieben. Die aus Rinderretina nach der Methode von Camps et al. (Nature 360 (1992) 684-686) gereinigten Gₜ-Proteine wurden mit den Lipidvesikeln für 5 min (Gₜßγ) oder 20 min (Gₜßγ plus Gₜα-GDP) auf Eis inkubiert, bevor das Enzym zugegeben wurde.

Bindungsstudien mit der rekombinanten PI3Kγ zeigen, daß das Regulatorprotein Ras in seiner aktiven GTP-beladenen Form mit dem Enzym assoziiert. Ras ist in seiner konstitutiv aktiven Version in der Lage, Zellen onkogen zu transformieren. Damit ergibt sich ein weiterer Hinweis für die potentielle Bedeutung von PI3Kγ bei der Regulation der zellulären Proliferation.

## Patentansprüche

1. Protein mit Phosphatidylinositol-3-Kinase-Aktivität
dadurch gekennzeichnet,
daß es
(a) die in SEQ ID NO. 2 dargestellte Aminosäuresequenz,
(b) die in SEQ ID N0. 4 dargestellte Aminosäuresequenz oder
(c) Varianten der Sequenz aus (a) mit einer Homologie von mindestens 80% zu der in SEQ ID NO. 2 oder SEQ ID NO. 4 dargestellten Aminosäuresequenz umfaßt.

2. Protein nach Anspruch 1,
dadurch gekennzeichnet,
daß es aus dem Menschen erhältlich ist.

3. Nucleinsäure,
dadurch gekennzeichnet,
daß sie für eine Phosphatidylinositol-3-Kinase nach Anspruch 1 oder 2 kodiert.

4. Nucleinsäure nach Anspruch 3,
dadurch gekennzeichnet,
daß sie ein rekombinantes DNA-Molekül ist.

5. Nucleinsäure nach Anspruch 3,
dadurch gekennzeichnet,
daß sie
(a) die in SEQ ID NO. 1 dargestellte Protein-kodierende Sequenz,
(b) die in SEQ ID NO. 3 dargestellte Protein-kodierende Sequenz,
(c) eine der Sequenz aus (a) oder (b) im Rahmen der Degeneration des genetischen Codes entsprechende Nucleinsäuresequenz oder
(d) eine mit den Sequenzen aus (a), (b) oder/und (c) unter stringenten Hybridisierungsbedingungen nach Waschen bei 55°C in einem wäßrigen Niedrigsalzpuffer hybridisierende Sequenz enthält.

6. Nukleinsäure,
**dadurch gekennzeichnet**,
dass sie einen mindestens 20 Nukleotide langen Abschnitt der in SEQ ID No. 1 oder SEQ ID No. 3 dargestellten Sequenz enthält, wobei dieser Abschnitt nicht die Sequenz TCC TGT GCA GGC TAC TGT GT ist.

7. Vektor,
dadurch gekennzeichnet,
daß er mindestens eine Kopie einer Nucleinsäure nach einem der Ansprüche 3 bis 6 oder einen Teil davon enthält.

8. Zelle,
dadurch gekennzeichnet,
daß sie mit einer Nucleinsäure nach einem der Ansprüche 3 bis 6 oder einem Vektor nach Anspruch 7 transformiert ist.

9. Verwendung eines Proteins nach Anspruch 1 oder 2 oder von Fragmenten dieses Proteins als Immunogen zur Herstellung von Antikörpern.

10. Antikörper gegen ein Protein nach Anspruch 1 oder 2,
dadurch gekennzeichnet,
daß er für PI3Kγ-spezifisch ist und keine Kreuzreaktion mit anderen Phosphatidylinositol-3-Kinasen zeigt.

11. Antikörper nach Anspruch 10,
dadurch gekennzeichnet,
daß er gegen eine Peptidsequenz gerichtet ist, die den Aminosäuren 741-745 der in SEQ ID NO. 2 dargestellten Aminosäuresequenz bzw. den Aminosäuren 742-746 der in SEQ ID NO. 4 dargestellten Aminosäuresequenz entspricht.

12. Verwendung einer Nucleinsäure nach einem der Ansprüche 3-7 zur Herstellung eines Mittels für die Gentherapie.

13. Pharmazeutische Zusammensetzung,
dadurch gekennzeichnet,
daß sie als aktiven Bestandteil ein Protein nach Anspruch 1 oder 2, einen Antikörper nach Anspruch 10 oder 11 oder eine Nucleinsäure nach einem der Ansprüche 3 bis 7 gegebenenfalls zusammen mit pharmazeutisch üblichen Hilfs-, Träger-, Füll- und Verdünnungsmitteln umfaßt.

14. Verfahren zur in vitro Bestimmung der Phosphatidylinositol-3-Kinase-Aktivität in Zellen, insbesondere von humanen Zellen,
**dadurch gekennzeichnet,**
daß man die Expressionsstärke des für ein Protein nach Anspruch 1 oder 2 codierenden Gens in der betreffenden Zelle ermittelt.

15. Verfahren nach Anspruch 14,
**dadurch gekennzeichnet,**
daß man die Expressionsstärke auf Transkript- oder/und Proteinebene ermittelt.

## Claims

1. Protein with phosphatidylinositol-3-kinase activity
wherein,
it comprises
(a) the amino acid sequence shown in SEQ ID NO. 2,
(b) the amino acid sequence shown in SEQ ID NO. 4 or
(c) variants of the sequence from (a) with a homology of at least 80 % to the amino acid sequence shown in SEQ ID NO.2 or SEQ ID NO.4.

2. Protein as claimed in claim 1,
wherein
it can be obtained from humans.

3. Nucleic acid,
wherein
it codes for a phosphatidylinositol-3-kinase as claimed in claim 1 or 2.

4. Nucleic acid as claimed in claim 3,
wherein
it is a recombinant DNA molecule.

5. Nucleic acid as claimed in claim 3,
wherein
it contains
(a) the protein-coding sequence shown in SEQ ID NO. 1
(b) the protein-coding sequence shown in SEQ ID NO. 3
(c) a nucleic acid sequence corresponding to the sequence from (a) or (b) within the scope of the degeneracy of the genetic code or
(d) a sequence which hybridizes with the sequences from (a), (b) or/and (c) under stringent hybridization conditions after washing at 55°C in an aqueous low salt buffer.

6. Nucleic acid,
wherein
it contains an at least 20 nucleotide long section of the sequence shown in SEQ ID NO. 1 or SEQ ID NO. 3 and this section is not the sequence TCC TGT GCA GGC TAC TGT GT.

7. Vector,
wherein
it contains at least one copy of a nucleic acid as claimed in one of the claims 3 to 6 or a part thereof.

8. Cell,
wherein
it is transformed by a nucleic acid as claimed in one of the claims 3 to 6 or by a vector as claimed in claim 7.

9. Use of a protein as claimed in claim 1 or 2 or of fragments of this protein as an immunogen for the production of antibodies.

10. Antibody against a protein as claimed in claim 1 or 2,
wherein
it is specific for PI3Kγ and does not exhibit any cross-reaction with other phosphatidylinositol-3-kinases.

11. Antibody as claimed in claim 10,
wherein
it is directed towards a peptide sequence which corresponds to the amino acids 741-745 of the amino acid sequence shown in SEQ ID NO. 2 or to the amino acids 742-746 of the amino acid sequence shown in SEQ ID NO. 4.

12. Use of a nucleic acid as claimed in one of the claims 3-7 for the production of an agent for gene therapy.

13. Pharmaceutical composition,
wherein
it comprises as the active component a protein as claimed in claim 1 or 2, an antibody as claimed in claim 10 or 11 or a nucleic acid as claimed in one of the claims 3 to 7 optionally together with common pharmaceutical auxiliary agents, carriers, fillers or diluents.

14. Method for the in vitro determination of phosphatidylinositol-3-kinase activity in cells and in particular in human cells,
wherein
the strength of the expression of the gene coding for a protein as claimed in claim 1 or 2 is determined in the respective cell.

15. Method as claimed in claim 14,
wherein
the strength of the expression is determined at a transcript or/and protein level.

## Revendications

1. Protéine ayant une activité de phosphatidylinositol-3-kinase, caractérisée en ce qu'elle contient
(a) la séquence d'aminoacides représentée par SEQ ID N° 2,
(b) la séquence d'aminoacides représentée par SEQ ID N° 4 ou
(c) des variantes de la séquence de (a) avec une homologie d'au moins 80 % avec la séquence d'aminoacides représentée par SEQ ID N° 2 ou SEQ ID N° 4.

2. Protéine selon la revendication 1, caractérisée en ce qu'elle peut être obtenue à partir de l'homme.

3. Acide nucléique, caractérisé en ce qu'il code pour une phosphatidylinositol-3-kinase selon la revendication 1 ou 2.

4. Acide nucléique selon la revendication 3, caractérisé en ce qu'il s'agit d'une molécule d'ADN recombiné.

5. Acide nucléique selon la revendication 3, caractérisé en ce qu'il contient:
(a) la séquence codant pour la protéine représentée par SEQ ID N° 1,
(b) la séquence codant pour la protéine représentée par SEQ ID N° 3,
(c) une séquence d'acide nucléique correspondant à la séquence de (a) ou (b) dans le cadre de la dégénérescence du code génétique, ou
(d) une séquence s'hybridant aux séquences de (a), (b), et/ou (c) dans des conditions d'hybridation stringentes après un lavage à 55°C dans un tampon aqueux à faible teneur en sels.

6. Acide nucléique, caractérisé en ce qu'il contient un fragment d'au moins 20 nucléotides de la séquence représentée par SEQ ID N° 1 ou SEQ ID N° 2, ce fragment n'étant pas la séquence TCC TGT GCA GGC TAC TGT GT.

7. Vecteur, caractérisé en ce qu'il contient au moins une copie d'un acide nucléique selon l'une des revendications 3 à 6 ou un de ses fragments.

8. Cellule caractérisée en ce qu'elle est transformée avec un acide nucléique selon l'une des revendications 3 à 6 ou avec un vecteur selon la revendication 7.

9. Utilisation d'une protéine selon la revendication 1 ou 2 ou de fragments de cette protéine comme immunogène pour la préparation d'anticorps.

10. Anticorps dirigé contre une protéine selon la revendication 1 ou 2, caractérisé en ce qu'il est spécifique de la PI3Kγ et ne présente pas de réaction croisée avec d'autres phosphatidylinositol-3-kinases.

11. Anticorps selon la revendication 10, caractérisé en ce qu'il est dirigé contre une séquence peptidique correspondant aux aminoacides 741-745 de la séquence d'aminoacides représentée par SEQ ID N° 2 ou aux aminoacides 742-746 de la séquence d'aminoacides représentée par SEQ ID N° 4.

12. Utilisation d'un acide nucléique selon l'une des revendications 3 à 7 pour la préparation d'un agent destiné à la thérapie génique.

13. Composition pharmaceutique, caractérisée en ce qu'elle contient comme ingrédient actif une protéine selon la revendication 1 ou 2, un anticorps selon la revendication 10 ou 11 ou un acide nucléique selon l'une des revendications 3 à 7, éventuellement avec des agents auxiliaires, des supports, des charges et des diluants classiques en pharmacie.

14. Procédé de détermination *in vitro* de l'activité de phosphatidylinositol-3-kinase dans des cellules, en particulier dans des cellules humaines, caractérisé en ce que l'on détermine le niveau d'expression du gène codant pour une protéine selon la revendication 1 ou 2 dans les cellules en question.

15. Procédé selon la revendication 14, caractérisé en ce que l'on détermine le niveau d'expression sur le plan du produit de transcription et/ou de la protéine.
